# EUROPÄISCHE PATENTSCHRIFT

(11) **EP 0 428 656 B1**
(45) Veröffentlichungstag und Bekanntmachung des Hinweises auf die Patenterteilung: **10.08.1994**
(21) Anmeldenummer: 90907060.9
(22) Anmeldetag: 14.05.1990
(51) Int. Cl.: C12N 5/08

(54) **HUMANE ZELLINIE LC5 SOWIE DEREN VERWENDUNG**
HUMAN CELL LINE LC5 AND ITS USE
LIGNEE CELLULAIRE HUMAINE LC5 ET SON UTILISATION

(30) Priorität: 18.05.1989 DE 3916251
(43) Veröffentlichungstag der Anmeldung: 29.05.1991
(73) Patentinhaber: GSF - Forschungszentrum für Umwelt und Gesundheit GmbH, 85764 Oberschleissheim (DE)
(72) Erfinder: ERFLE, Volker, D-8000 München 80 (DE); MELLERT, Werner, D-8044 Lohhof (DE)
(74) Vertreter: Klunker . Schmitt-Nilson . Hirsch
(86) Internationale Anmeldenummer: EP9000774
(87) Internationale Veröffentlichungsnummer: WO9014419

(56) Entgegenhaltungen:
- Federation Proceedings, vol. 19, no. 15, 1960 E.V. Davis et al.: "Continuous cell cultivation of isogenous cell lines from thehuman embryo", Seite 386

## Beschreibung

Diese Erfindung betrifft die humane Zellinie LC5 sowie deren Verwendung zur Untersuchung der Replikationshemmung für Retroviren. Insbesondere befaßt sich diese Erfindung mit der Untersuchung von Hemmsubstanzen und neutralisierenden Antikörpern für Immundefizienzviren des Menschen (HIV) und der Primaten (SIV) unter Verwendung dieser humanen Zellinie LC5.

Die in-vitro-Untersuchung von Hemmsubstanzen für Retroviren hat in den letzten Jahren insbesondere auf dem Gebiet der Aids-Forschung an Bedeutung gewonnen. Ziel dieser Forschung ist es, eine Therapie für HIV-infizierte Menschen zu entwickeln. Die Untersuchungen auf neutralisierende Antikörper sind insbesondere im Hinblick auf Prognosekriterien bei Verlaufsuntersuchungen von HIV-Infizierten und bei der Herstellung von Impfstoffen interessant. Bei beiden Fragestellungen - sowohl Therapie als auch Impfung - ist eine möglichst einfache Quantifizierung der Menge an infektiösen Viruseinheiten erforderlich.

Für die in-vitro-Untersuchung wurden bisher für Retroviren empfängliche Zellinien verwendet, deren Zellen in einer Suspension wachsen. Eine derartige Suspensionslösung mit der Zellinie, die mit Retroviren, beispielsweise HIV-1-Stämmen, infiziert ist, kann nicht ohne weiteres abgesaugt bzw. abpipettiert werden, da die Zellen in der Suspension schwimmen und somit zusammen mit der Suspensionslösung abgesaugt werden können. Daher muß die Suspension einige Zeit stehengelassen werden, bis sich die infizierten Zellen abgesetzt haben, so daß die sich darüber befindliche Suspensionslösung entnommen werden kann. Aber selbst dann ist es immer noch möglich, daß einzelne Zellen durch das Absaugen wieder aufgewirbelt und somit zusammen mit der Suspensionslösung entnommen werden. Daher führt die Auswertung, wieviele Zellen von den Retroviren infiziert worden sind, zu ungenauen Untersuchungsergebnissen. Außerdem ist eine derartige Vorgehensweise mit einem erheblichen Arbeits- und Zeitaufwand verbunden.

Die große Anzahl von in Frage kommenden Seren und Substanzen, die sich als Hemmsubstanzen eignen können, macht ein schnelles und verläßliches Screening-Verfahren notwendig. Dies kann beispielsweise dadurch erreicht werden, daß es automatisiert werden kann. Die Verwendung von Zellinien in Suspensionen erlaubt wegen der oben genannten Nachteile keine Automatisierung des Verfahrens.

Der Erfindung liegt daher die Aufgabe zugrunde, eine Zellinie anzugeben, die für Retroviren empfänglich ist und die eine schnelle und zuverlässige Untersuchung von Seren und Substanzen hinsichtlich ihrer Eignung als Hemmsubstanzen für Retroviren zuläßt. Insbesondere soll eine kosten- und zeitsparende Automatisierung einer solchen Untersuchung ermöglicht werden.

Die Aufgabe wird erfindungsgemäß durch die Zellinie LC5 gelöst, die für Retroviren, insbesondere für Immundefizienzviren des Menschen (HIV) und des Primaten (SIV) empfänglich ist und mit diesen Viren infiziert werden kann. Die erfindungsgemäße humane Zellinie LC5 eignet sich somit insbesondere zur Untersuchung von Hemmsubstanzen für derartige Retroviren. Erfindungsgemäß wird daher die Verwendung dieser neuen Zellinie LC5 zur Untersuchung von neutralisierenden Seren und Hemmsubstanzen für Retroviren vorgeschlagen. Die Erfindung wird durch die Merkmale der Unteransprüche weitergebildet.

Die Zellinie LC5 stammt aus dem Lungengewebe eines menschlichen Embryos (E.V. Dantes and V.S. Bolin Fed. Proc. 19, 386, 1960). Die ursprüngliche humane Zelllinie wird unter der Bezeichnung L 132 bei der American Type Culture Collection (ATCC, Nr. CCL-5) aufbewahrt. Die Zellinie L 132 wurde mehrfach kultiviert, mit Antibiotika gegen Mykoplasmen behandelt und subkloniert, wodurch die Zellinie LC5 erhalten wurde.

Die erfindungsgemäße Zellinie LC5 wurde am 09. März 1989 bei der Collection Nationale de Cultures des Microorganismes (CNCM), Paris, unter der Hinterlegungsnummer I-842 hinterlegt.

Die erfindungsgemäße humane Zellinie LC5 wächst adhärent an Kunststoff- und Glasoberflächen, weshalb die Untersuchung von Hemmsubstanzen für Retroviren problemlos automatisiert werden kann. Dadurch, daß die Zellinie adhärent an Kunststoff- oder Glasoberflächen wächst, besteht keine Gefahr mehr, daß beim Absaugen bzw. Abpipettieren der Suspensionslösung in der Lösung schwimmende Zellen mit abgesaugt werden können. Somit ist insbesondere für den Absaugvorgang eine Vollautomatisierung möglich.

Die Zellen der Zellinie LC5 wachsen als Monolayer-Kulturen in RPMI 1640 (Gibco) als Kulturmedium, das mit 10 % fetalem Kälberserum bei 37° C und 5 % CO₂ versetzt ist. Die Morphologie der Zellen ist epitheloid. Die Zellen exprimieren typische Merkmale mesenchymaler Zellen wie Laminin, Fibronektin und Vimentin und außerdem geringe Mengen von Zytokeratin.

Die Erfindung wird nachfolgend unter Bezugnahme auf die Zeichnungen näher erläutert. In den Zeichnungen zeigt:
- Fig. 1: eine mikroskopische Darstellung der LC5-Zellen nach Infektion mit HIV-1 und Anfärbung der HIV-1 Proteinzellen (Kolonie);
- Fig. 2: eine Korrelation des HIV-Titers mit der Anzahl von HIV-Protein-positiven Kolonien bzw. des Farbumschlages im Überstand;
- Fig. 3: eine elektronenmikroskopische Darstellung der Freisetzung von HIV-Partikeln in der Zellinie LC5-HIV;
- Fig. 4: die Reduktion von HIV-positiven LC5-Kolonien nach der Behandlung durch die Dideoxycytidin (ddC); und
- Fig. 5: die Reduktion von HIV-positiven LC5-Kolonien durch Neutralisation von HIV mittels eines Humanserums.

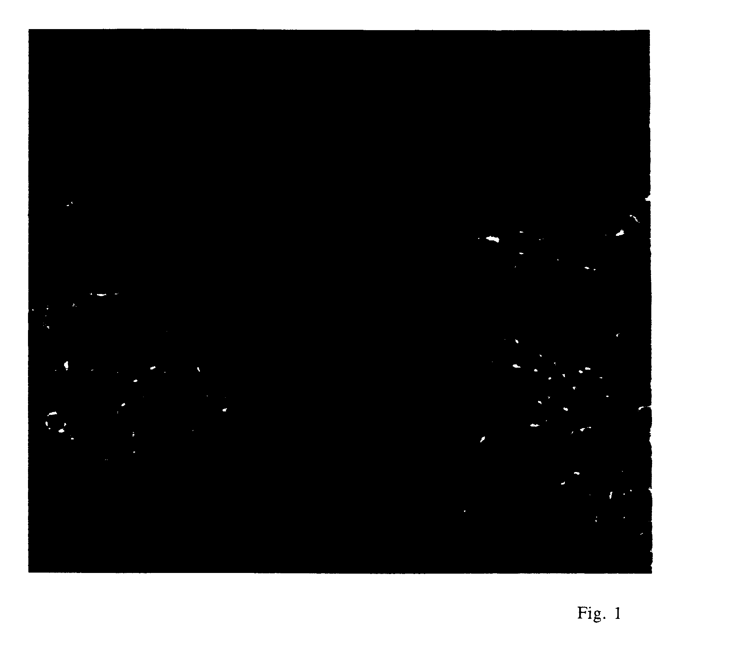

Zur Verwendung der humanen Zellinie LC5 für die in-vitro-Untersuchung von Kemmsubstanzen beispielsweise für HIV wurde die humane Zellinie LC5 zunächst mit verschiedenen HIV-1 Stämmen (HTLV IIIb, RF, Rutz) und mit HIV-2 infiziert. Dies führte nach 3-4 Tagen zur Ausbildung von Kolonien, die aus 4-8 Zellen bestanden, die HIV-Strukturproteine produzierten. Der Nachweis der HIV-Strukturproteinproduktion erfolgte mit einem serologischen Test unter Verwendung von Antikörpern gegen diese Proteine (1. Antikörper) und mit einem gegen das entsprechende Immunglobulin gerichteten Substrat, das an Peroxidase oder Phosphatase gekoppelte Antikörper (2. Antikörper) enthält. Dabei wurde 3-Aminoethylcarbazol, das wasserunlöslich ist, als spezifisches Substrat verwendet. Dieses Verfahren ist bekannt als sogenannte indirekte Immunperoxidasefärbung; vgl. dazu Mellert et al. AlFOl, 105-107, 1986. Die Anzahl der gefärbten Kolonien wurde unter dem Lichtmikroskop bestimmt. Die Fig. 1 zeigt die mit HIV-1 infizierten Zellen der humanen Zellinie LC5, die nach diesem Färbeverfahren angefärbt worden sind.

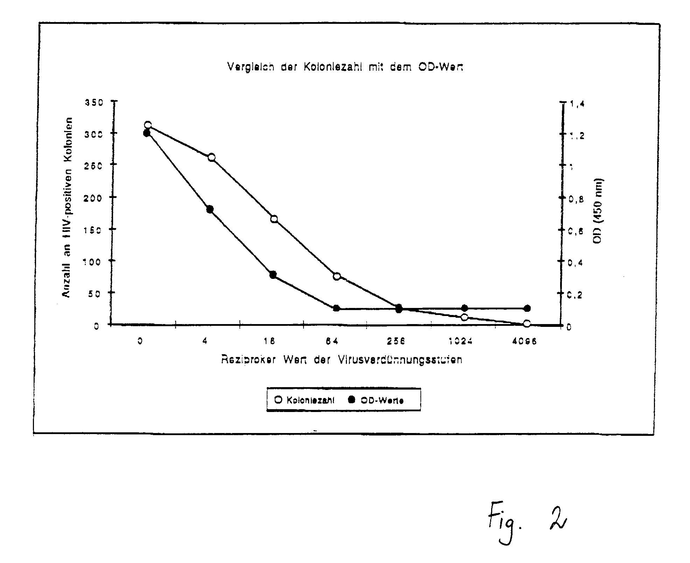

Wie aus Fig. 2 ersichtlich ist, ist die Anzahl der gebildeten HIV-Protein produzierenden Kolonien direkt abhängig von der Anzahl der infektiösen HIV-Partikel. Zur Ermittlung dieser direkten Proportionalität wurden unterschiedliche Viruskonzentrationen für die Infizierung mit der humanen Zellinie LC5 verwendet. In Fig. 2 ist die Anzahl an HIV-positiven Kolonien über dem reziproken Wert der verwendeten Virusverdünnungsstufen angegeben. Bei Verwendung eines löslichen Substrats, beispielsweise Orthophenylendiamin (OD) kann anstelle der angefärbten Zellen oder Kolonien ein Farbumschlag in der Lösung über den Zellen induziert werden. Die Intensität des Farbumschlags ist direkt proportional zu der Anzahl der infektiösen HIV-Partikel, mit der die humane Zellinie LC5 infiziert ist. Dies ist ebenfalls aus Fig. 2 ersichtlich.

Die erfindungsgemäße humane Zellinie LC5 wurde mit HIV-1 infiziert und nach der Infektion durch Einzelzellverdünnung kloniert. Die HIV-positiven Zellen wurden dann zu einer Zellinie auswachsen gelassen.

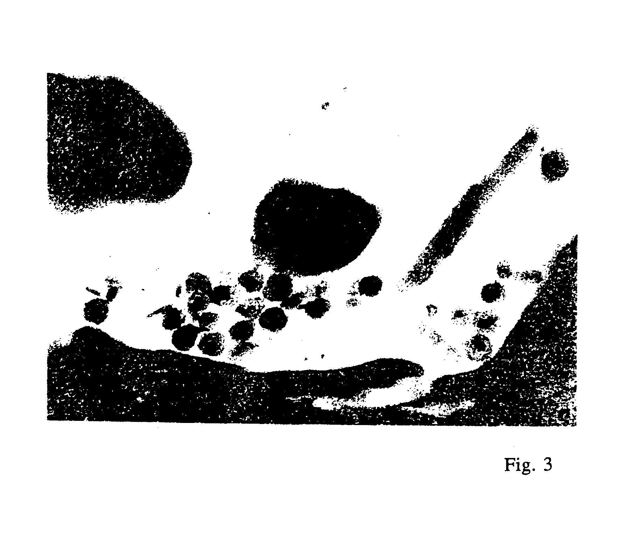

Diese Zellinie trägt die Bezeichnung LC5-HIV und wurde ebenfalls bei CNCM in Paris unter der Hinterlegungsnummer I-843 am 09. März 1989 hinterlegt. Die Zelline LC5-HIV produziert infektiöse Partikel in das umgebende Medium im Umfang von ca. 10⁵ infektiösen Partikeln pro Milliliter Medium. In Fig. 3 ist eine elektonenmikroskopische Darstellung der Freisetzung von HIV-Partikeln in der Zellinie LC5-HIV mit einer Vergrößerung von 80000 dargestellt.

### A. Testung von Hemmsubstanzen

Das erfindunsgemäße Verfahren zur Testung von Hemmsubstanzen für Retroviren unter Verwendung der humanen Zellinie LC5 und der Zellinie LC5-HIV wurde am Beispiel HIV folgendermaßen durchgeführt.

Die HIV-infizierten LC5-HIV-Zellen wurden etwa 1 Woche lang behandelt, dann erfolgte die Testung der infektiösen Einheiten im Kulturüberstand durch eine Neuinfektion von nichtinfizierten LC5-Zellen mit nachfolgender immunhistochemischer Bestimmung der Anzahl an HIV-positiven Kolonien. Als Virus produzierende Zellen wurden HIV-infizierte humane fetale Lungenzellen LC5-HIV, und als nichtinfizierte Zellen wurden humane fetale Lungenzellen LC5 verwendet. Beide Zelllinien wurden mit RPMI 1640 (Gibco) und 10 % fetalem Kälberserum (FKS) bei 37° C und 5 % CO₂ kultiviert.

Als positive Kontrolle eines anti-HIV-wirksamen Therapeutikums wurde Dideoxycytidin (ddC) verwendet. Diese Substanz wurde in unterschiedlichen Konzentrationen mit einer entsprechenden Verdünnungsreihe im Bereich von 20 µg/ml bis 0,2 ng/ml verwendet.

Die Behandlung der virusproduzierenden Zellen wurde folgendermaßen durchgeführt. In 24-Loch Testplatten (Nunc) wurden 3 x 10³ LC5-HIV-Zellen in 1 ml Kulturmedium (RPMI 1640/10 % FKS) eingesät und innerhalb einer Stunde mit den unterschiedlichen Konzentrationen des Therapeutikums Dideoxycytidin versetzt (Tag 0). Für jede Testkonzentration wurden vier Kulturen angesetzt. Danach wurden die Zellen eine Woche lang bei 37° C und 5 % CO₂ bebrütet, wobei an Tag 3 und 5 erneut das Therapeutikum zum Kulturmedium zugegeben wurde. An Tag 7 wurden die Platten 10 Minuten lang bei 3000 rpm zentrifugiert, und pro Vertiefung der Testplatten wurden 200 µl Überstand abpipetiert.

Zur Quantifizierung der infektiösen Einheiten im Überstand wurden in 96-Mikrotiter-Testplatten jeweils 2 x 10³ LC5-Zellen in 100 µl Medium eingesät und drei Stunden lang bei 37° C und 5 % CO₂ bebrütet. Das Medium wurde abgesaugt, und pro Vertiefung wurden 100 µl Kulturüberstand der behandelten LC5-HIV-Zellen gegeben, der aus den 24-Loch Testplatten entnommen wurde, wobei pro Vertiefung dieser 24-Loch Testplatten jeweils zwei Kulturen in den 96-Mikrotiter-Testplatten angesetzt wurden. Der Überstand wurde eine Stunde auf den Zellen belassen, dann abgesaugt und durch 200 µl frisches Kulturmedium ersetzt. Die Testplatten wurden drei Tage lang bei 37° C und 5 % CO₂ bebrütet. Die Bestimmung der Anzahl an HIV-positiven Kolonien erfolgte durch die indirekte Immunperoxidasefärbung. Dazu wurden die Zellen mit Methanol/Aceton (Verhältnis 1:1, -20° C) fünf Minuten lang fixiert und an der Luft getrocknet. Der Nachweis der HIV-Strukturproteine erfolgte mittels eines HIV-Antikörper-positiven humanen Referenzserums (1:150 in PBS (Phosphat gepufferte Kochsalzlösung) verdünnt) sowie Sichtbarmachung des Antigen-Antikörper-Komplexes durch an Meerrettichperoxidase gekoppeltes Kaninchen-anti-human-IgG unter Verwendung von 3-Aminoethylcarbazol/H₂O₂ als Substrat. Die Anzahl der gefärbten Kolonien wurde unter dem Lichtmikroskop bestimmt.

Es zeigte sich, daß die Kontrollsubstanz Dideoxycytidin (ddC) bei 20 µg/ml für die LC5-HIV-Zellen nicht toxisch war. Die Anzahl der HIV-positiven LC5-Kolonien, die durch Infektion mit dem vorbehandelten Kulturüberstand gebildet wurden, ist aus Tabelle 1 zu entnehmen. Die dort angegebenen Werte stellen jeweils einen Mittelwert aus je acht Meßwerten dar. In der nachfolgenden Tabelle ist ebenfalls die Standardabweichung SD angegeben, die aus jeweils acht Meßwerten pro Konzentration ermittelt wurde.

**Tab. 1**

| Anzahl an HIV-positiven Kolonien nach Infektion von LC5-Zellen mit Überstand von ddC-behandelten LC5-HIV-Zellen | | | | | | | |
|---|---|---|---|---|---|---|---|
| | 20µg | 2 µg | 200ng | 20ng | 2ng | 0,2ng | 0 ng/ml |
| ddC:MW | 0 | 0 | 3,9* | 11,0 | 21,9 | 18,8 | 130,4 |
| SD | | | 1,9 | 3,0 | 4,6 | 6,6 | 21,1 |
| MW = Mittelwert SD = Standardabweichung | | | | | | | |

| | | | | | | | |
|---|---|---|---|---|---|---|---|
| * = Anzahl an HIV-pos. LC5-Kolonien | | | | | | | |

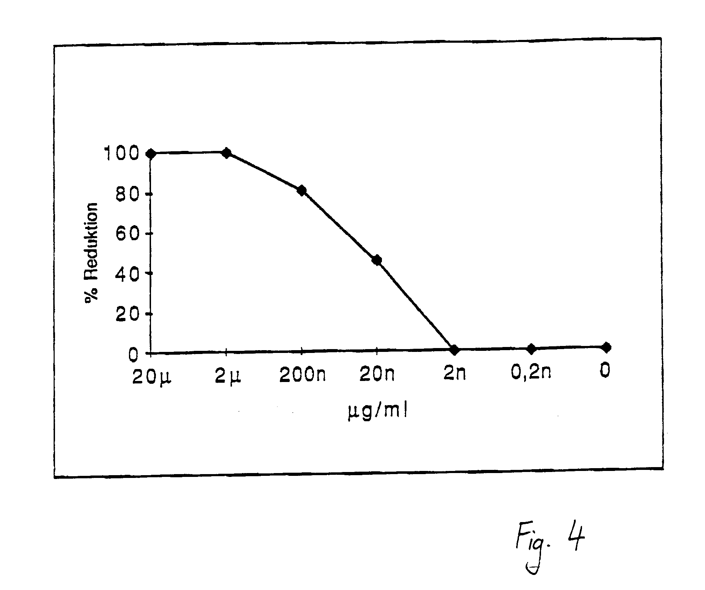

Die Umrechnung der Koloniezahl in Prozent Reduktion im Vergleich zu einer unbehandelten Kontrolle ergibt eine Reduktion der infektiösen Einheiten, wie in Fig. 4 dargestellt ist. Aus Fig. 4 ist ersichtlich, daß die Behandlung von LC5-HIV-Zellen mit Dideoxycytidin bis zu einer Konzentration von 2 µg/ml eine 100%-ige Reduktion der infektiösen Einheiten im Kulturüberstand zur Folge hat.

Aufgrund dieser Ergebnisse kann davon ausgegangen werden, daß durch die Behandlung von HIV-infizierten humanen, fetalen Lungenzellen LC5-HIV und durch die Bestimmung der infektiösen Einheiten im Kulturüberstand mit LC5-Zellen als Targetzellen die anti-HIV-Wirksamkeit unterschiedlicher Substanzen beurteilt werden kann. Dieses System erfaßt alle möglichen Angriffspunkte eines Therapeutikums (Virusanheftung, Virusfreisetzung, Inaktivierung freier Viruspartikel) und ist somit als Screening-System geeignet. Durch die Verwendung der Monolayer-Zellen sind alle Verfahrensschritte mit Hilfe eines XYZ-Roboters vollautomatisierbar.

### B. Testung neutralisierender Antikörper

Das erfindungsgemäße Verfahren zur Testung neutralisierender Antikörper unter Verwendung der humanen Zellline LC5 sowie LC5-HIV wurde am Beispiel HIV-1 folgendermaßen durchgeführt.

Als zellfreie HIV Suspension wurde Zellkulturüberstand von LC5-HIV verwendet, der eine Woche nach Passage der Zellen geerntet worden war. Der überstand wurde 10 Minuten bei 1000 g zentrifugiert und mittels eines Ultrafilters (Porengröße 45 µm) filtriert. Zu 50 µl dieses Überstandes wurden 50 µl des Testserums in unterschiedlichen Konzentrationen gegeben und eine Stunde bei Raumtemperatur inkubiert. Die Quantifizierung der nach dieser Inkubation verbliebenen infektiösen Einheiten wurde durch Beimpfung von LC5 Zellen mit dem Serum-Virusgemisch nach der unter Punkt A beschriebenen Methode durchgeführt.

Es zeigte sich, daß in Gegenwart von neutralisierenden Antikörpern die Anzahl der HIV-positiven LC5-Kolonien mit der Serumverdünnung korrelierte (Tabelle 2). Die in der Tabelle 2 angegebenen Werte stellen jeweils einen Mittelwert aus je vier Meßwerten dar. Daneben ist die Standardabweichung (SD) angegeben, die aus diesen vier Werten errechnet worden ist.

**Tabelle 2**

| Anzahl an HIV-positiven LC5-Kolonien in Gegenwart von neutralisierenden Antikörpern | | | | | |
|---|---|---|---|---|---|
| reziproke Serumverdünnung | 10 | 20 | 40 | 80 | 160 |
| pos. Serum | 0 | 0 | 15(± 6) | 93(± 14) | 201(± 29) |
| neg. Serum | 187(± 16) | 224(± 32) | 218(± 28) | 230(± 23) | 219(± 34) |

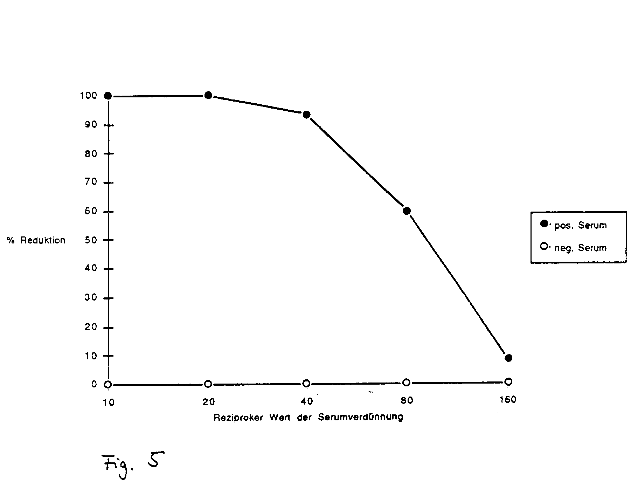

Die Umrechnung der Koloniezahl in Prozent Reduktion im Vergleich zu einer unbehandelten Kontrolle ergibt eine Reduktion der infektiösen Einheiten, wie in Fig. 5 dargestellt ist. Aus Fig. 5 ist ersichtlich, daß neutralisierende Seren eine Reduktion der Koloniezahl um bis zu 100 % zur Folge haben. Aufgrund dieser Ergebnisse kann davon ausgegangen werden, daß durch Infektion von LC5-Zellen die neutralisierende Eigenschaft von Seren (Reduktion der Infektiosität von HIV) beurteilt werden kann.

## Patentansprüche

1. Humane Zellinie LC5 (C N C M I-842)

2. Humane Zellinie LC5 gemäß Anspruch 1,
dadurch gekennzeichnet,
daß sie mit Retroviren infiziert ist.

3. Humane Zellinie LC5 gemäß Anspruch 2,
dadurch gekennzeichnet,
daß die Retroviren immundefiziente Viren des Menschen (HIV) und der Primaten (SIV) sind.

4. Humane Zellinie LC5 gemäß den Ansprüchen 1 bis 3,
dadurch gekennzeichnet,
daß sie mit HIV-1 Stämmen und/oder mit HIV-2 Stämmen infiziert ist.

5. Humane Zellinie LC5 gemäß den Ansprüchen 1 bis 4,
dadurch gekennzeichnet,
daß die Zellinie LC5-HIV ist.

6. Humane Zellinie LC5 gemäß den Ansprüchen 1 bis 5,
dadurch gekennzeichnet,
daß sie adhärent an Kunststoff- und/oder Glasoberflächen wächst.

7. Verwendung der humanen Zellinie LC5 gemäß den Ansprüche 1 bis 6 zur Untersuchung von Hemmsubstanzen für Retroviren.

8. Verwendung der humanen Zellinie LC5 gemäß Anspruch 7,
dadurch gekennzeichnet,
daß die Hemmsubstanzen neutralisierende Antikörper für Retroviren sind.

9. Verwendung der humanen Zellinie LC5 gemäß den Ansprüchen 7 und 8,
dadurch gekennzeichnet,
daß die Hemmsubstanzen neutralisierende Antikörper für Immundefizienzviren des Menschen (HIV) und Primaten (SIV) sind.

10. Verwendung der humanen Zellinie LC5 gemäß den Ansprüchen 1 bis 9 zur Untersuchung von Hemmsubstanzen und neutralisierenden Seren für Retroviren,
gekennzeichnet durch
- Behandlung von mit Retroviren infizierten LC5-Zellen durch Einsäen der infizierten Zellinie in ein Kulturmedium, insbesondere in ein RPMI 1640, das 10 % fetales Kälberserum enthält, und Zugabe eines anti-Retrovirus-wirksamen Therapeutikums;
- Bebrüten dieser Zellen für einen bestimmten Zeitraum, insbeonsdere Bebrüten der Zellen über einen Zeitraum von einer Woche bei 30°C und 5 % CO²;
- Quantifizierung der infektiösen Einheiten im Überstand über den Zellen mit Hilfe der humanen Zellinie LC5; und
- Bestimmung der Anzahl an Retrovirus-positiven Kolonien, insbesondere mit Hilfe einer indirekten Immunperoxidosefärbung.

11. Verwendung der humanen Zellinie LC5 gemäß Anspruch 10,
dadurch gekennzeichnet,
daß das Retrovirus ein Immundefizienzvirus des Menschen (HIV) ist.

## Claims

1. A human cell line LC5 ( C N C M I - 842).

2. The human cell line LC5 of claim 1, characterized in that it is infected with retroviruses.

3. The human cell line LC5 of claim 2, characterized in that the retroviruses are immunodeficient viruses of humans (HIV) and primates (SIV).

4. The human cell line LCS of claims 1 to 3, characterized in that it is infected with HIV-1 strains and/or with HIV-2 strains.

5. The human cell line LC5 of claims 1 to 4, characterized in that the cell line is LC5-HIV.

6. The human cell line LC5 of claims 1 to 5, characterized in that it grows adhering to plastic and/or glass surfaces.

7. Use of the human cell line LC5 of claims 1 to 6 for testing inhibitors for retroviruses.

8. The use of the human cell line LC5 of claim 7, characterized in that the inhibitors are neutralizing antibodies for retroviruses.

9. The use of the human cell line LC5 of claims 7 and 8, characterized in that the inhibitors are neutralizing antibodies for immunodeficient viruses of humans (HIV) and primates (SIV).

10. The use of the human cell line LC5 of claims 1 to 9 for testing inhibitors and neutralizing serums for retroviruses, characterized by
treating LC5 cells infected with retroviruses by inoculating the infected cell line in a culture medium, in particular in an RPMI 1640 containing 10% fetal calf serum, and adding an anti-retroviral therapeutic agent;
incubating these cells for a certain period, in particular incubating the cells over a period of one week at 30°C and 5% CO²;
quantifying the infectious units in the supernatant over the cells with the aid of the human cell line LC5; and
determining the number of retrovirus-positive colonies, in particular with the aid of an indirect immunoperoxidase staining.

11. The use of the human cell line LC5 of claim 10, characterized in that the retrovirus is an immunodeficient virus of humans (HIV).

## Revendications

1. Lignée cellulaire humaine LC5 (CNCM I-842).

2. Lignée cellulaire humaine LC5 selon la revendication 1, caractérisée en ce qu'elle est infectée par des rétrovirus.

3. Lignée cellulaire humaine LC5 selon la revendication 2, caractérisée en ce que les rétrovirus sont des virus du syndrome immunodéficitaire acquis de l'homme (HIV) et des primates (SIV).

4. Lignée cellulaire humaine LC5 selon les revendications 1 à 3, caractérisée en ce qu'elle est infectée par des souches d'HIV-1 et/ou des souches d'HIV-2.

5. Lignée cellulaire humaine LC5 selon les revendications 1 à 4, caractérisée en ce qu'elle est la lignée cellulaire LC5-HIV.

6. Lignée cellulaire humaine LC5 selon les revendications 1 à 5, caractérisée en ce qu'elle se développe sous une forme adhérant à des surfaces de matière plastique ou de verre.

7. Utilisation de la lignée cellulaire LC5 selon les revendications 1 à 6 pour l'étude des substances inhibant les rétrovirus.

8. Utilisation de la lignée cellulaire humaine LC5 selon la revendication 7, caractérisée en ce que les substances inhibitrices sont des anticorps neutralisants pour les rétrovirus.

9. Utilisation de la lignée cellulaire humaine LC5 selon les revendications 7 et 8, caractérisée en ce que les substances inhibitrices sont des anticorps neutralisants pour les virus du syndrome immunodéficitaire acquis de l'homme (HIV) et des primates (SIV).

10. Utilisation de la lignée cellulaire humaine LC5 selon les revendications 1 à 9 pour l'étude de substances inhibitrices et des sérums neutralisants pour les rétrovirus, caractérisée en ce que :
- on traite des cellules LC5 infectées par des rétrovirus par ensemencement de la lignée cellulaire infectée dans un milieu de culture, en particulier dans un RPMI 1640 qui contient 10 % de sérum de veau foetal, et addition d'un médicament actif contre les rétrovirus ;
- on fait incuber ces cellules pendant un temps déterminé, en particulier pendant une semaine à 30 °C, et avec 5 % de CO₂ ;
- on quantifie les unités infectieuses dans le liquide surnageant sur les cellules à l'aide de la lignée cellulaire LC5 ; et
- on détermine le nombre de colonnes positives en ce qui concerne les rétrovirus, en particulier à l'aide d'une coloration à l'immunoperoxydase indirecte.

11. Utilisation de la lignée cellulaire humaine LC5 selon la revendication 10, caractérisée en ce que le rétrovirus est un virus du syndrome immunodéficitaire acquis (HIV) de l'homme.
